(19) Europäisches Patentamt
European Patent Office
Office européen des brevets

(11) Numéro de publication : 0 390 905 B1

# (12) FASCICULE DE BREVET EUROPEEN

(45) Date de publication du fascicule du brevet : **11.08.93 Bulletin 93/32**

(21) Numéro de dépôt : **89911568.7**

(22) Date de dépôt : **02.10.89**

(86) Numéro de dépôt international : **PCT/FR89/00505**

(87) Numéro de publication internationale : **WO 90/03966 19.04.90 Gazette 90/09**

(51) Int. Cl.$^5$ : **C07C 237/10,** C07C 311/09, C11D 1/92, A62D 1/02, B01F 17/22

## (54) COMPOSES AMIDOFLUORES, LEUR OBTENTION ET LEUR UTILISATION.

(30) Priorité : **10.10.88 FR 8813613**

(43) Date de publication de la demande : **10.10.90 Bulletin 90/41**

(45) Mention de la délivrance du brevet : **11.08.93 Bulletin 93/32**

(84) Etats contractants désignés : **AT CH DE GB IT LI NL SE**

(56) Documents cités :
**FR-A- 2 376 208**
**CHEMIKER-ZEITUNG, vol. 104, no. 2, février 1980, Heidelberg, DE; J. N. MEUSSDOERFFER et al: "Fluortenside und fluorhaltige Phobiermittel - grenzflächenaktive Perfluorverbindungen"**

(73) Titulaire : **SZONYI, Istvan**
**120, Rue de Roquebillière**
**F-06300 Nice (FR)**
Titulaire : **SZONYI, Stéphane**
**26, avenue de Grande Bretagne**
**MC-98000 Monte Carlo (MC)**
Titulaire : **SZONYI, François**
**26, avenue de Grande Bretagne**
**MC-98000 Monte Carlo (MC)**

(72) Inventeur : **SZONYI, Istvan**
**120, Rue de Roquebillière**
**F-06300 Nice (FR)**
Inventeur : **SZONYI, Stéphane**
**26, avenue de Grande Bretagne**
**MC-98000 Monte Carlo (MC)**
Inventeur : **SZONYI, François**
**26, avenue de Grande Bretagne**
**MC-98000 Monte Carlo (MC)**

EP 0 390 905 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne de nouveaux tensioactifs amidofluorés exempts de dérivés des protéines, caractérisés par le fait qu'ils contiennent un radical perfluoroalkyl, ayant 4 à 20 atomes de carbone et une ou plusieurs liaisons peptidiques symbolisées par -CONH-, issues d'une aminolyse d'une fonction ester carboxylique.

La plupart des tensioactifs fluorés se trouvant actuellement sur le marché sont issus des deux grandes familles de base perfluoroalkylée suivantes :

$$R_FSO_2F \text{ et } R_FC_2H_4\text{-}I$$

$R_F$ représente une chaîne perfluoroalkylée linéaire ou ramifiée.

Les procédés d'obtention de ces bases sont connus depuis de nombreuses années, et les premiers brevets sont déjà tombés dans le domaine public.

Il est bien connu que l'élaboration des tensioactifs fluorés à partir de ces produits de départ, peut poser des problèmes techniques d'ordre divers, comme par exemple la séparation des sous produits et des composés réactifs en excés ou le retraitement des solvants etc. Ce qui finalement augmente le prix de revient des tensioactifs obtenus.

Pour palier à ces inconvénients, il a été mis au point et c'est ce qui constitue la nouveauté principale de la présente invention, des nouveaux tensioactifs,composés amidofluorés, préparés à l'aide d'une méthode simple et originale, ne produisant aucun déchet ou sous produit, ou solvant à retraiter. C'est un procédé à "une cuve" (one pot reaction).

Le procédé selon l'invention consiste dans le fait que les sous produits sont transformés au cours du traitement et condensés avec le produit principal Ainsi les excés sont "digérés" ne laissant aucun déchet.

Par exemple à partir du composé $R_FSO_2F$ et d'une diamine, on obtient le produit intermédiaire décrit dans le brevet US 2759019/56 :

$$R_FSO_2NH\text{-}\ C_3H_6N(CH_3)_2.$$

Pour sa préparation au lieu d'utiliser le diéthyléther comme solvant, on utilise selon l'invention l'amine en excés , et le surplus d'amine sera "digéré" par un traitement ultérieur ; par exemple par adjonction d'un halogénure d'acide alcanoique, les réactions sont les suivantes :

$$R_FSO_2NHC_3H_6N(CH_3)_2 + ClCH_2\text{-}COO\ Et \rightarrow R_FSO_2\text{-}NH\text{-}C_3H_6\ \overset{+}{N}\ (CH_3)_2CH_2\text{-}COOEt \qquad Cl^-$$

L'amine en excés réagit également avec le monochloro acétate d'éthyle formant le composé suivant :

$$NH_2\text{-}C_3H_6\text{-}\ \overset{+}{N}\ (CH_3)_2\text{-}CH_2\text{-}COOEt \qquad Cl^-$$

Dans une deuxième série de réactions qui se font presque instantanément l'ester subit une aminolyse en milieu anhydre, ce qui conduit aux produits de condensation peptidique suivants :

$$R_FSO_2NH\text{-}C_3H_6\underset{Cl^-}{\overset{+}{N}}(CH_3)_2\text{-}CH_2COOEt\ \ Cl^- + NH_2\text{-}C_3H_6\text{-}\underset{Cl^-}{\overset{Cl^-\,+}{N}}(CH_3)_2\text{-}CH_2\text{-}COOEt \longrightarrow$$

$$\longrightarrow R_FSO_2\text{-}NH\text{-}C_3H_6\text{-}\overset{+}{N}\text{-}(CH_3)_2\text{-}CH_2\text{-}CONH\text{-}C_3H_6\overset{+}{N}(CH_3)_2\text{-}CH_2\text{-}COOEt$$

En fonction de la température, du dosage et du temps d'adjonction du monochloroacétate d'éthyle, on obtient un mélange de tensioactifs fluorés plus ou moins évolués vers la polycondensation ou vers la substitution de l'atome d'hydrogène lié à l'atome d'azote :

$$R_FSO_2NHC_3H_6\underset{Cl^-}{\overset{+}{N}}(CH_3)_2CH_2\text{-}CO\text{-}\left[NH\text{-}C_3H_6N(CH_3)_2\text{-}CH_2\text{-}CO\right]_n O\text{-}Et$$

$$R_FSO_2\underset{CH_2\text{-}CO..}{N}C_3H_6\overset{Cl^-\,+}{N}(CH_3)_2\text{-}CH_2\text{-}CO\text{-}\underset{CO\text{-}CH_2..}{N}\text{-}C_3H_6\text{-}\overset{Cl^-\,+}{N}(CH_3)_2CH_2\text{-}CO...$$

Par ailleurs l'utilisation comme base de départ du composé : $R_FC_2H_4$-I pose un autre problème . Il est bien

connu que son amination directe donne de très mauvais rendements, il y a production d'un taux important de produits d'élimination qui est l'alcène correspondant. Pour cette raison, certains fabricants font intervenir dans le procédé une sulfonation qui est évidemment très laborieuse et couteuse. On peut éviter la formation d'alcène en employant un excés d'amine, ce qui nous conduit au produit suivant :

$$R_FC_2H_4NHC_3H_6\text{-}N(CH_3)_2$$

Ensuite, la réaction d'un monohalogénure d'acétate d'éthyle sur le produit principal et sur l'excés d'amine produit les composés suivants :

$$R_FC_2H_4NHC_3H_6\overset{+}{N}(CH_3)_2\text{-}COOEt \quad Cl^-$$

et

$$NH_2\text{-}C_3H_6\text{-}\overset{+}{N}(CH_3)_2CH_2\text{-}COOEt \quad Cl^-$$

Ces produits réagissant à leur tour par aminolyse, selon l'invention on obtient les substances suivantes :

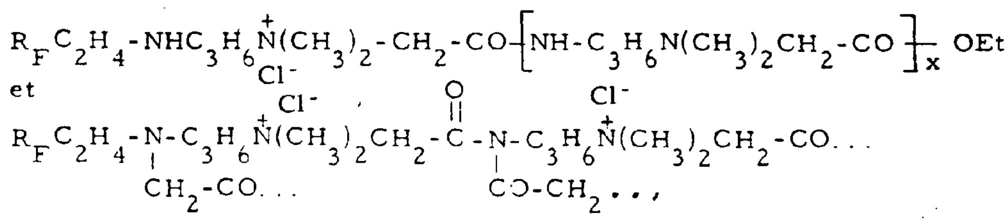

On peut utiliser d'autres amines et aussi d'autres bases de départ comme par exemple le composé $R_F(CH_2)_q\text{-}SO_2\text{-}X$ ou $R_F(CH_2)_q\text{-}COX$, sans sortir du cadre de l'invention.

X est un halogénure

**q** est un nombre entier de 0 à 6

Ces polyamides fluorés obtenus selon l'invention contiennent encore un groupement ester. On hydrolyse par la soude ou la potasse caustique en quantité suffisante, pour obtenir les sels correspondants.

Les exemples qui suivent illustrent l'invention et montrent comment elle peut être mise en pratique, mais ces exemples ne sont nullement limitatifs.

Exemple n°1 :

Dans un réacteur, muni d'un réfrigérant à reflux et d'un agitateur on introduit 61,2 pp (0,6 mole) de dimethylamino propylamine, on chauffe à 60-70°C et on ajoute lentement 100,4 pp (0,2 mole) de fluorure de perfluorooctylsulfonyle. La réaction est exothermique, on maintient la température à 80-95°C pendant l'addition et ensuite on mélange encore pendant deux heures à 80-90°C. (pp = partie en poids)

On laisse refroidir le mélange à 70-80°C et on ajoute en petites portions 73, 5pp (0, 6 mole) de monochloracétate d'éthyle en agitant. La réaction est exothermique ; on empèche la température de monter au delà de 115°C.

On laisse tourner le mélange à 80°C pendant 15 heures, ensuite on ajoute 300 pp d'eau, on mélange 15 minutes et on ajoute une solution contenant 66 pp (1,07 mole ) de potasse caustique technique et 120 pp d'eau. La température est maintenue à 80-82°C en mélangeant pendant 6 heures.

Le mélange obtenu pèse 721 pp. (pp = partie en poids)

Après repos, sa densité à 24°C est de 1,167 et son pH 10, 8. Il contient environ 25% de matière active.

| Résultats des mesures | | |
|---|---|---|
| teneur en matière active des solutions (%) | tension superficielle dynes/cm | tension interfaciale dynes/cm |
| 0,1 | 14,9 | 4,2 |
| 0,01 | 15,0 | - |
| 0,001 | 17,5 | - |

test de Ross and Miles:
Solution contenant 0, 025% de matière active
Hauteur de mousse mesurée à la température de 25°C :

| $t_o$ | $t_{5mn}$ |
|---|---|
| 196 mm | 160 mm |

Exemple n°2 : On dissout dans 52,8 pp d'eau 10 pp d'urée, 20 pp de butyldiglycol, 10 pp de laurylamidobétaïne (30%), 1,7 pp d'octyl sulfate de soude (40%) et 4,5 pp de produit obtenu dans l'exemple n° 1 et on ajuste le pH à 8.

La solution obtenue est un émulseur concentré utilisable à la dilution de 15 à 20 fois son poids. Ce type d'émulseur est connu sous le nom d'AFFF: Agent Formant un Film Flottant. (pp = partie en poids)

| Résultats des mesures de la solution 6% | |
|---|---|
| Tension superficielle (dynes/cm) | Tension interfaciale (dynes/cm) |
| 15,2 | 1,5 |

Résultats d'essais d'extinction sur feu d'heptane et réallumage selon la norme AFNOR S-60-210. Pour une solution 6%:

Extinction 99 % 60 secondes
Extinction 100 % 100 secondes
Réallumage 100 % 660 secondes

Exemple n°3

Dans un réacteur , muni d'un réfrigérant à reflux et d'un agitateur, on verse 122,4 pp (1, 2 mole) de dimethylaminopropylamine, on chauffe à 60-70°C et on ajoute en plusieurs portions 94,8 pp (0,2 mole) de iodure de perfluorohexylethane.

On maintient la température entre 80 et 90°C pendant l'addition et ensuite on mélange encore pendant trois heures à 90°C. Puis on laisse descendre la température à 70°C et on ajoute en plusieurs petites portions 147 pp (1,2 mole) de monochloracétate d'éthyle en agitant. La réaction est exothermique et on veille à ce que la température ne dépasse pas 115°C.

On laisse tourner le mélange à 80°C pendant 15 heures, ensuite on ajoute 250 pp d'eau, on mélange 15 minutes et on verse une solution contenant 103 pp (1,67mole) de potasse caustique technique et 200 pp d'eau. La température est maintenue à 80-82°C en mélangeant pendant 6 heures.

Le mélange obtenu pèse 917 pp. Il contient environ 25% de matière active. (pp = partie en poids)

| Résultats des mesures | | |
|---|---|---|
| Teneur en matière active des solutions (%) | tension superficielle (dynes/cm) | tension interfaciale (dynes/cm) |
| 0,1 | 16,7 | 5 |
| 0,01 | 19,0 | - |
| 0,001 | 25,2 | - |

Exemple n°4 :

On dispose 30 pp de solution colloidale de gomme Xanthane traitée selon l'exemple n° 6 du brevet MC n° 1939 et on ajoute 20 pp de laurylsulfate de triéthanolamine à 40%, 5 pp d'octyl sulfate de soude à 40%, 8 pp de butyldigly-col, 10 pp d'urée et 3,3 pp de surfactant à 25 % obtenu selon l'exemple n°3.On complète le mélange par 23,7 pp d'eau.

La solution concentrée obtenue est un émulseur polyvalent utilisé pour combattre les feux d'hydrocarbures et les feux de liquides'polaires. Ce type de produit est connu sous le nom d'APPPP ou A4P: Agent Produisant une Pellicule Protectrice Polyvalente. (pp = partie en poids)

Elle est utilisée en diluant six parties de concentré dans 94 parties d'eau, pour les essais suivants:

Tension superficielle : 18 dynes/cm

Tension interfaciale sur cyclohexane : 2 dynes/cm

Extinction sur feu d'heptane

(norme AFNOR S-60-210)

99% ........ 70 secondes

100% ........ 120 secondes

Réallumage 100 % ........ 720 secondes

Extinction sur feu d'acétone

(norme AFNOR S-60-201)

99% ........ 40 secondes

100% ........ 60 secondes

Réallumage 100% ........ 1 200 secondes

Exemple 5:

On fait réagir, dans un réacteur muni d'un réfrigérant à reflux et d'un agitateur, 122,4 pp (1,2 mole) de dimethylaminopropylamine avec 114,4 pp (0,2 mole) d'un mélange d'iodure de perfluoroalkyléthane chauffé préalablement à 30°C, répondant à la formule $C_nF_{2n}C_2H_4I$ et contenant environ (% poids ) :

54% $C_6F_{13}$-$C_2H_4I$

27 % $C_8F_{17}$-$C_2H_4I$

11 % $C_{10}F_{21}$ -$C_2H_4I$

4,5% $C_{12}F_{25}$-$C_2H_4I$

2% $C_{14}F_{29}$- $C_2H_4I$

1% $C_{16}F_{33}$-$C_2H_4I$

La température est maintenue à 90°C en agitant pendant 4 heures. Ensuite on laisse refroidir à 70°C et on ajoute en plusieurs portions 147 pp (1,2 mole) de monochloroac étate d'éthyle en mélangeant. La réaction est exothermique, et on règle la vitesse d'addition de telle façon que la température reste au dessous de 115°C. Après addition complète, on mélange à 80-85°C pendant 15 heures, Ensuite, on ajoute 250 pp d'eau et on mélange un quart d'heure. On verse une solution préalablement préparée, contenant 103 pp (1,67mole) de potasse caustique technique et 200 pp d'eau. On continue de mélanger à 80-82°C pendant 6heures. (pp = partie enpoids) Le mélange obtenu pèse 944 pp. Il contient environ 25% de matière active

| Résultats des mesures | | |
|---|---|---|
| teneur en matière active des solutions (%) | tension superficielle en dynes/cm | tension interfaciale sur cyclohexane (dynes/cm) |
| 0,1 | 17,2 | 5,2 |
| 0,01 | 20,0 | - |
| 0,001 | 24,0 | - |

Exemple n° 6 :

On exécute la même préparation décrite dans l'exemple n°4, mais à la différence qu'au lieu d'utiliser 3, 3 pp de surfactant à 25% obtenu suivant l'exemple n°3 on utilise le surfactant préparé selon l'exemple n° 1.

Les résultats obtenus avec une solution à 6% sont les suivants :

Tension superficielle : 16,0 dynes/cm

Tension interfaciale sur cyclohexane : 2,0 dynes/cm

Extinction d'un feu d'heptane

(norme AFNOR S-60-210)

99% ............ 50 secondes

100 % ............ 90 secondes

Réallumage 100% ............ 800 secondes

Extinction d'un feu d'acétone

(norme AFNOR S-60-201)

99% ............ 30 secondes

100% ............ 45 secondes

Réallumage ............ 1 200 secondes

## Revendications

1. Nouveaux tensioactifs amidofluorés exempts de dérivés des protéines, caractérisés par le fait qu'ils contiennent un radical perfluoroalkyl, ayant 4 à 20 atomes de carbone et une ou plusieurs liaisons peptidiques symbolisées par -CONH-, issues d'une aminolyse d'une fonction ester carboxylique, répondant à la formule générale suivante :

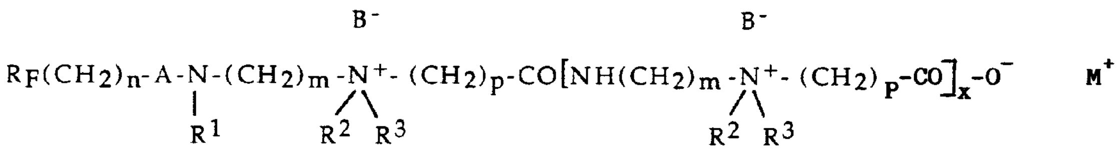

| | |
|---|---|
| RF | : groupement perfluoroalkyl contenant 4 à 20 atomes de carbone, |
| A | : $SO_2$ ou CO ou néant, |
| n | : un nombre entier de 0 à 3, |
| m | : un nombre entier de 3 à 6, |
| p | : un nombre entier de 1 à 2, |
| x | : un nombre entier de 1 à 6, |
| $R^1$ | : un atome d'hydrogène ou un groupement alkyle substitué ou un groupement alcanacyl substitué, |
| $R^2$ et $R^3$ | : deux groupements alkyles mineurs différents ou identiques, |
| $B^-$ | : un ion halogène, |
| $M^+$ | : un ion métal alcalin. |

2. Nouveaux procédés de fabrication de tensioactifs amidofluorés selon la revendication (1), caractérisés par le fait que l'excés d'amine utilisé au départ comme solvant est consommé par la suite, grâce à sa réaction avec un halogénure d'ester alcanoïque;d'une part les réactions de substitution de l'halogénure d'ester alcanoïque par une diamine et/ou une diamine substituée et/ou perfluoroalkylée,et d'autre part l'aminolyse des esters, permettent au procédé de n'avoir aucun déchet ou solvant à séparer.

3. Nouveaux procédés de fabrication de tensioactifs amidofluorés selon la revendication (2), caractérisés par le fait que le monohalogénure d'ester alcanoïque et le monochlorure d'acétate d'éthyle.

4. Nouveaux procédés de fabrication de tensioactifs amidofluorés, selon les revendications (2) et (3), caractérisés par le fait que l'halogénure d'ester alcanoïque transformé en aminoester par une diamine, de préférence par la diméthylamino propylamine, réagit sur l'aminoester perfluoroalkylé par aminolyse.

5. Utilisation des composés fluorés selon la revendication (1) dans différents domaines de la technique pour diminuer la tension superficielle de l'eau ou des huiles et en particulier dans la composition de mousses extinctrices pour la lutte contre l'incendie.

**Patentansprüche**

1. Neue grenzflächenaktive mit Amidfluor versetzte Stoffe, frei von Proteinderivaten, die sich durch die Tatsache auszeichnen, daß sie ein Radikal Perfluoroalkyl mit 4 bis 30 Kohlenstoffatomen und eine oder mehrere durch - CONH- dargestellte peptidische Verbindungen besitzen, entstanden aus einer Aminolyse einer Ester-Carboxylfunktion, die durch folgende allgemeine Formel dargestellt wird:

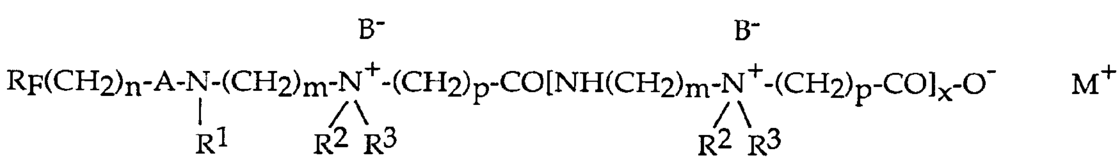

| | |
|---|---|
| $R_F$: | Perfluoroalkyl-Gruppierung mit 4 bis 20 Kohlenstoffatomen, |
| A: | $SO_2$ oder CO oder nichts, |
| n: | eine ganze Zahl zwischen 0 und 3, |
| m: | eine ganze Zahl zwischen 3 und 6, |
| p: | eine ganze Zahl zwischen 1 und 2, |
| x: | eine ganze Zahl zwischen 1 und 6, |

| | |
|---|---|
| $R^1$: | ein Wasserstoffatom oder eine substituierte Alkyl-Gruppierung oder eine substituierte Acyl-Alkan-Gruppierung, |
| $R^2$ & $R^3$: | zwei kleinere unterschiedliche oder gleiche Alkyl-Gruppierungen, |
| $B^-$: | ein Halogenion, |
| $M^+$: | ein alkalisches Metallion. |

**2.** Neue Fabrikationsverfahren von grenzflächenaktiven mit Amidfluor versetzten Stoffen gemäß Patentanspruch (1), gekennzeichnet durch die Tatsache, daß das überschüssige Amin, das zu Beginn als Lösungsmittel verwendet wird, nachher dank seiner Reaktion mit einem Halogenid aus alkanischem Ester verbraucht wird; einerseits die Substitutionsreaktionen des Halogenids aus alkanischem Ester durch ein Diamin und/oder ein substituiertes und/oder perfluoralkylisiertes Diamin, und andererseits die Amynolyse der Ester, die es ermöglichen, daß das Verfahren kein Abfallprodukt oder Lösungsmittel abscheidet.

**3.** Neue Fabrikationsverfahren von grenzflächenaktiven mit Amidfluor versetzten Stoffen gemäß Patentanspruch (2), gekennzeichnet durch die Tatsache, daß das Monohalogenid aus alkanischem Ester das Monochlorid aus Ethylazetat ist.

**4.** Neue Fabrikationsverfahren von grenzflächenaktiven mit Amidfluor versetzten Stoffen gemäß Patentanspruch (2) und (3), gekennzeichnet durch die Tatsache, daß das durch ein Diamin, vorzugsweise durch das Dimethylaminopropylamin in Aminoester verwandelte Halogenid aus alkanischem Ester auf das perfluoralkylisierte Aminoester durch Aminolyse reagiert.

**5.** Nutzung der fluorisierten Bestandteile gemäß Patentanspruch (1) in verschiedenen technischen Bereichen zur Verringerung der Oberflächenspannung von Wasser oder Ölen besonders in der Zusammenstellung von Löschschäumen gegen Brände.

## Claims

**1.** New amidofluorinated surfactants, containing no protein derivatives, characterized by the presence of a perfluoroalkyl radical, with 4 to 20 carbon atoms and one or several peptide bonds symbolized by -CONH-, resulting from aminolysis of a carboxyl ester function, corresponding to the following general formula:

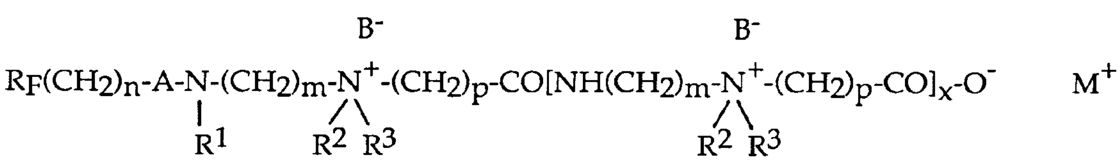

| | |
|---|---|
| $R_F$: | perfluoroalkyl group containing 4 to 20 carbon atoms, |
| A: | $SO_2$ or CO or nothing, |
| n: | an integer from 0 to 3, |
| m: | an integer from 3 to 6, |
| p: | an integer from 1 to 2, |
| x: | an integer from 1 to 6, |
| $R^1$: | a hydrogen atom or substituent alkyl group or substituent acyl alkanes group, |
| $R^2$ & $R^3$: | two different or identical minor alkyl groups, |
| $B^-$: | a halogen ion, |
| $M^+$: | an alkaline metal ion. |

**2.** New manufacturing process for amidofluorinated surfactants according to claim (1), characterized by the face that the excess amine used from the start as a solvent is subsequently consumed by reacting with an alkanoic ester halide; on one hand the alkanoic ester halide substitution reactions by a diamine and/or substituent and/or perfluoralkylated diamine, and on the other by aminolysis of esters, so the process leaves no residue or solvent for separation.

**3.** New manufacturing process for amidofluorinated surfactants according to claim (2), characterized by the

fact that the alkanoic ester monohalide is ethyl acetate monochloride.

4. New manufacturing process for amidofluorinated surfactants according to claims (2) and (3), characterized by the fact that the alkanoic ester halide transformed into an aminoester by a diamine, preferably dimethylaminopropylamine, reacts with the perfluoroalkylated aminoester by aminolysis.

5. Use of fluorinated compounds according to claim (1) in various applications to reduce surface tension of water or oils, in particular in the composition of foam for fire extinguishers.